(19) Europäisches Patentamt
European Patent Office
Office européen des brevets

(11) Publication number: **0 088 615**
**B1**

(12) **EUROPEAN PATENT SPECIFICATION**

(45) Date of publication of patent specification: **06.08.86**

(51) Int. Cl.[4]: **C 07 C 69/54,** C 07 C 67/317

(21) Application number: **83301194.3**

(22) Date of filing: **07.03.83**

(54) **Process for the preparation of alpha, beta unsaturated acid esters.**

(30) Priority: **08.03.82 US 355603**

(43) Date of publication of application:
**14.09.83 Bulletin 83/37**

(45) Publication of the grant of the patent:
**06.08.86 Bulletin 86/32**

(84) Designated Contracting States:
**AT BE CH DE FR GB IT LI LU NL SE**

(56) References cited:
**US-A-3 855 279**
**US-A-4 029 695**

(73) Proprietor: **CHEM SYSTEMS, Inc.**
**747 Third Avenue**
**New York New York 10017 (US)**

(72) Inventor: **Gelbein, Abraham P.**
**45 Headley Road**
**Morristown Morris New Jersey 07960 (US)**

(74) Representative: **Harrison, Michael Robert et al**
**URQUHART-DYKES & LORD 5th Floor Tower House Merrion Way**
**Leeds LS2 8PA West Yorkshire (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 088 615 B1**

**Description**

Background of the disclosure

Field of the invention

The instant invention is directed to a method for converting a carboxylic acid ester to its corresponding alpha, beta unsaturated ester by means of an oxidative dehydrogenation procedure which is characterized by the absence of significant amounts of the corresponding saturated and alpha, beta unsaturated acids in the product stream. More specifically, the instant invention is directed to a process for converting the carboxylic acid ester, methylisobutyrate, into its corresponding alpha, beta unsaturated ester, methylmethacrylate.

Background of the prior art

There are many processes described in the prior art directed to methods of converting a carboxylic acid ester into its corresponding alpha, beta unsaturated ester by means of an oxidation process. These methods are characterized by their unique catalyst systems.

U.S. Patent Specification No. 3855279 describes a process in which acids or esters may be dehydrogenated in the presence of oxygen and a catalyst which is a calcined residue of the mixed phosphates of iron and lead. There is disclosed in U.S. Patent Specification No. 4029695 a similar process in which the catalyst further includes a modifier component selected from manganese, uranium, praseodymium, calcium, strontium and chromium.

Of these processes for converting carboxylic acid esters into the corresponding alpha, beta unsaturated ester, the most important specific reaction is the conversion of methylisobutyrate (MIB) into methylmethacrylate (MMA). These reactions, unfortunately, are characterized by the formation of the undesirable by-products, methylacrylic acid (MAA) and isobutyric acid (IBA).

The reactions that occur in a typical prior art process involving the oxidation of MIB into MMA in the vapor phase are as follows:

(1) $H_3C—CH(COOCH_3)CH_3+\frac{1}{2}O_2 \rightarrow H_2C=CH(COOCH_3)CH_3+H_2O$
(MIB) (MMA)

(2) $H_3C—CH(COOCH_3)CH_3+H_2O \rightarrow H_3C—CH(COOH)CH_3+CH_3OH$
(IBA)

(3) $H_2C=CH(COOCH_3)CH_3+H_2O \rightarrow H_3C—CH(COOH)CH_3+CH_3OH$
(MAA)

The latter two reactions, reactions 2 and 3, are undesirable hydrolysis reactions.

The formation of these unwanted acids, IBA and MAA, can be separated from the desired, product, methylmethacrylate. However, this operation is very expensive owing to the high capital cost of building the units necessary to effectuate this separation. Moreover, there are significant energy costs associated with separating the acids from the ester. In addition, the acids must be converted back to the corresponding saturated esters and thereafter recycled to the oxidative dehydrogenation reactor.

Summary of invention

A major problem associated with the formation of alpha, beta unsaturated esters by conversion of the corresponding carboxylic acid ester through an oxidative dehydrogenation procedure has now been overcome. It has now been found that the formation of the undesirable by-products, the corresponding saturated acid and the corresponding alpha, beta unsaturated acid, can be eliminated. This is effectuated by adding methanol to the reactants introduced into the oxidative reactor. The addition of the methanol effectively eliminates the formation of the undesirable corresponding saturated and alpha, beta unsaturated acids.

This process has particular application to the specific carboxylic acid ester, methylisobutyrate, which is converted to the corresponding alpha, beta unsaturated ester, methylmethacrylate. The addition of methanol effectively eliminates the formation of the undesirable saturated acid, isobutyric acid, and the corresponding alpha, beta unsaturated acid, methacrylic acid.

In accordance with the present invention there is provided a process for converting a carboxylic acid ester into a corresponding alpha, beta unsaturated ester, the process characterised in that the carboxylic acid ester is methylisobutyrate and the process comprises reacting said methylisobutyrate, in the vapor phase, with oxygen gas, in the presence of gaseous methanol, said methanol present in a molar concentration at least equal to that of said carboxylic acid ester, to form methylmethacrylate.

Detailed description

The current invention is directed to a process for forming an alpha, beta unsaturated ester by the conversion of the corresponding carboxylic acid ester in the presence of methanol. More specifically, the instant invention is directed to a process in which methylisobutyrate (MIB) is converted into

methylmethacrylate (MMA) by reacting the MIB in the vapor phase with oxygen. In this process gaseous methanol is introduced into the reactor along with the MIB. The effect of this reaction is to substantially eliminate the formation of undesirable by-products, the corresponding saturated acid and the corresponding alpha, beta unsaturated acid, which in the preferred species, results in the elimination of the formation of isobutyric acid (IBA) and methacrylic acid (MAA) respectively.

The methanol is preferably added to the reactor downstream of the MIB inlet. It is emphasized, however, that the process of this invention contemplates also, as an alternate preferred embodiment, the introduction of the methanol directly into the MIB feed. Introduction of the methanol downstream of the point of introduction of the MIB is preferred since the methanol is thus introduced at a point subsequent to the initiation of reaction between the oxygen and the methylisobutyrate. This minimizes methanol losses due to minimization of the reaction between methanol and oxygen.

Another advantage of introducing the methanol downstream of the point of introduction of the methylisobutyrate is the availability of employing different catalysts for the oxidative dehydrogenation reaction and esterification reaction, which suppresses acid formation. The catalyst employed in this reaction can be contained in series in the same reactor vessel or in separate vessels connected in series. Suitable catalysts for the esterification reaction include iron phosphorous oxides, other supported and unsupported heteropoly acid oxides, activated aluminas, silica alumina, transition metal oxides, such as tungsten oxide, chromia, molecular sieves and the like.

The operating condition under which the reaction occurs is a function of the type of catalyst employed. In general, the temperature of reaction may range between about 200°C and 500°C. More preferably, the temperature is in the range of between about 300°C and 400°C. The pressure under which the reaction occurs is in the range of between about 0.1 and 10 atmospheres. The time of reaction, that is, the residence time, is in the range of between about 0.1 and 10 seconds. The molar ratio of methanol to methylisobutyrate is in the range of between about 1 and 20 moles of methanol per mole of methylisobutyrate. More preferably, the methanol to methylisobutyrate ratio is in the range of between about 1 and 10 moles of methanol per mole of methylisobutyrate. The process of this invention, the gaseous oxydehydrogenation reaction of methylisobutyrate to methylmethacrylate can be carried out in a fixed bed, fluidized bed or transport reactor system.

The following examples are given to illustrate the scope and spirit of the instant invention.

Example 1

The following feed composition:

| | |
|---|---|
| Methylisobutyrate | 2.2 mole percent |
| Methanol | 15.4 mole percent |
| Oxygen | 1.8 mole percent |
| Nitrogen | 80.6 mole percent |

was introduced into a tubular stainless steel reactor containing 10.5 grams of an iron-phosphorous oxide catalyst. The temperature of the reactor was controlled by a liquid heat transfer medium into which the reactor was immersed. The space velocity of the gaseous reactants was 1,700 $hour^{-1}$, measured at standard temperature and pressure. A temperature of 360°C was maintained in the reactor.

The products of this reaction were measured at various times during the continuous operation of this reaction. Table 1 below summarizes the analysis of the reactants at three specific time periods during the operation of this reaction.

TABLE 1

| Hours on stream | MIB conv., % | Selectivity*, mole % MMA | MAA | IBA | $CO_x$ |
|---|---|---|---|---|---|
| 1 | 65 | 51 | 0 | 0 | 11 |
| 2 | 61 | 52 | 0 | 0 | 11 |
| 3.5 | 55 | 52 | 0 | 0 | 11 |

*Unaccounted for materials are unidentified light end by-products.

It is additionally noted that surprisingly no significant quantity of methanol oxidation products were found in the analysis of the reaction products of this reaction.

Comparative Example 1

Example 1 was repeated with the single change that the feed composition during the running of this continuous operation did not include any methanol, which was present in the feed composition of Example 1 at a molar ratio of 7 moles of methanol per mole of methylisobutyrate. Thus, the feed composition in this comparative example was as follows:

| | |
|---|---|
| Methylisobutyrate | 2.2 mole percent |
| Oxygen | 1.8 mole percent |
| Nitrogen | 96.0 mole percent |

based on same ratio of the three constituents absent methanol.

The product stream of this reaction was sampled three times during the continuous operation of this reactor. The results of this analysis appear below in Table 2.

TABLE 2

| Hours on stream | MIB conv., % | Selectivity*, mole % MMA | MAA | IBA | $CO_x$ |
|---|---|---|---|---|---|
| 1 | 72 | 54 | 8 | 7 | 12 |
| 2.5 | 65 | 51 | 9 | 12 | 13 |
| 3.5 | 60 | 51 | 10 | 14 | 14 |

*Unaccounted for materials are unidentified light end by-products.

As can be seen by a comparison of Tables 1 and 2, representing the process of this invention and the process of the prior art respectively, no undesirable saturated acid, isobutyric acid (IBA), was formed in Example 1, representative of the process of this invention. IBA, however, was formed in Comparative Example 1, an example of the process of the prior art. Similarly, the process of the instant invention, illustrated in Example 1, did not result in the formation of any alpha, beta unsaturated acid, methacrylic acid (MAA). However methacrylic acid was formed in the process of the prior art, that is, in Comparative Example 1.

Example 2

Example 1 was repeated except that the feed ratio of methanol to methylisobutyrate was reduced from a molar ratio of 7 to a molar ratio of 2. This resulted in a feed composition as follows:

| | |
|---|---|
| Methylisobutyrate | 2.5 mole percent |
| Methanol | 5.0 mole percent |
| Oxygen | 1.8 mole percent |
| Nitrogen | 90.7 mole percent |

During the continuous operation of the reactor, product samples were obtained resulting in the analysis which is summarized in Table 3 below.

TABLE 3

| Hours on stream | MIB conv., % | Selectivity*, mole % MMA | MAA | IBA | $CO_x$ |
|---|---|---|---|---|---|
| 1 | 65 | 51 | 0 | 0 | 11 |
| 2 | 61 | 52 | 0 | 0 | 11 |
| 3.5 | 55 | 52 | 0 | 0 | 11 |

*Unaccounted for materials are unidentified light end by-products.

These results again indicate the surprisingly significant improvement in the suppression of the acid products, methacrylic acid (MAA) and isobutyric acid (IBA), with the addition of even a lesser excess of methanol to methylisobutyrate.

As in Example 1, no significant quantity of methanol oxidation products were found in the analysis of the reaction products of this reaction.

The above preferred embodiment and examples are given to illustrate the scope and spirit of this invention. They should not, however, be construed as limiting the invention to those embodiments and examples given hereinbefore. These embodiments and examples will suggest other embodiments and examples to those skilled in the art. These embodiments and examples are also within the scope and spirit of the instant invention. Thus, the instant invention should be limited only by the appended claims.

**Claims**

1. A process for converting a carboxylic acid ester into a corresponding alpha, beta unsaturated ester, the process characterised in that the carboxylic acid ester is methylisobutyrate and the process comprises reacting said methylisobutyrate, in the vapor phase, with oxygen gas, in the presence of gaseous methanol, said methanol present in a molar concentration at least equal to that of said carboxylic acid ester, to form methylmethacrylate.

2. A process according to claim 1 characterised in that said molar ratio of methanol to methylisobutyrate is in the range of between about 1 and 20.

3. A process according to claim 2 characterised in that said molar ratio of said methanol to methylisobutyrate is in the range of between about 1 and 10.

4. A process according to any of the preceding claims characterised in that said methanol is added to the reaction downstream of the point at which the methylisobutyrate is fed into the reactor.

5. A process according to any of the preceding claims characterised in that the reaction is catalyzed by a catalyst selected from iron-phosphorous oxides, other supported and unsupported heteropoly acid oxides, activated aluminas, silica alumina, transistion metal oxides, chromia and molecular sieves.

6. A process according to claim 5 characterised in that said catalyst is an iron-phosphorous oxide.

**Patentansprüche**

1. Verfahren zur Umwandlung eines Carbonsäureesters in den entsprechenden alpha-, betaungesättigten Ester, dadurch gekennzeichnet, daß man als Carbonsäureester Methylisobutyrat verwendet und dieses Methylisobutyrat durch Umsetzung in der Dampfphase mit Sauerstoffgas in Anwesenheit von gasförmigem Methanol, wobei das Methanol in einer Molkonzentration vorliegt, die wenigstens der Molkonzentration des Carbonsäureesters entspricht, in Methylmethacrylat überführt.

2. Verfahren nach Anspruch 1, dadurch gekennzeichnet, daß man bei einem Molverhältnis von Methanol zu Methylisobutyrat im Bereich zwischen etwa 1 und 20 arbeitet.

3. Verfahren nach Anspruch 2, dadurch gekennzeichnet, daß man bei einem Molverhältnis von Methanol zu Methylisobutyrat im Bereich zwischen etwa 1 und 10 arbeitet.

4. Verfahren nach irgendeinem der vorhergehenden Ansprüche, dadurch gekennzeichnet, daß man das Methanol stromabwärts von der Stelle zuführt, an welcher das Methylisobutyrat in den Reaktor eingespeist wird.

5. Verfahren nach irgendeinem der vorhergehenden Ansprüche, dadurch gekennzeichnet, daß man die Umsetzung in Gegenwart eines Katalysators durchführt, der ausgewählt ist aus Eisen-Phosphor-Oxiden, sonstigen getragenen oder nicht getragenen Heteropolysäureoxiden, aktivierten Aluminiumoxiden, Siliciumdioxid-Aluminiumoxid, Übergangsmetalloxiden, Chromoxid und Molekularsieben.

6. Verfahren nach Anspruch 5, dadurch gekennzeichnet, daß man als Katalysator ein Eisen-Phosphor-Oxid verwendet.

**Revendications**

1. Procédé pour la conversion d'un ester d'acide carboxylique en un ester alpha, bêta insaturé correspondant, le procédé étant caractérisé en ce que l'ester d'acide carboxylique est le méthylisobutyrate et le procédé comprend la réaction dudit méthylisobutyrate, en phase vapeur, avec de l'oxygène gazeux, en présence de méthanol gazeux, ledit méthanol étant présent en une concentration molaire au moins égale à celle dudit ester d'acide carboxylique, pour former le méthylméthacrylate.

2. Procédé selon la revendication 1, caractérisé en ce que le rapport molaire précité du méthanol au méthylisobutyrate est dans la gamme d'environ 1 à 20.

3. Procédé selon la revendication 2 caractérisé en ce que le rapport molaire précité du méthanol au méthylisobutyrate est dans la gamme d'environ 1 à 10.

4. Procédé selon l'une des revendications précédentes caractérisé en ce que le méthanol précité est ajouté à la réaction en aval du point auquel le méthylisobutyrate est alimenté dans le réacteur.

5. Procédé selon l'une des revendications précédentes caractérisé en ce que la réaction est catalysée par un catalyseur choisi parmi les oxydes de fer-phosphore, d'autres oxydes d'hétéropoly acide supportés

et non supportés, alumines activées, silice alumine, oxydes de métaux de transition, chrome et tamis moléculaires.

6. Procédé selon la revendication 5 caractérisé en ce que le catalyseur précité est un oxyde de fer-phosphore.